# EUROPEAN PATENT APPLICATION

(11) **EP 3 037 515 A1**
(43) Date of publication of application: **29.06.2016**
(21) Application number: 15202502.9
(22) Date of filing: 23.12.2015
(51) Int. Cl.: C12M 1/42, C12N 15/87, C12N 5/07, C12N 13/00, C12M 3/06

(54) **MODIFIED CELL PREPARED BY PUTTING MATERIAL INTO THE CELL WITHOUT USING DELIVERY VEHICLE**

(30) Priority: 28.12.2014 KR 20140191302; 14.12.2015 KR 20150178234
(71) Applicant: Femtofab Co., Ltd., Pohang-si, Gyeongsangbuk-do (KR)
(72) Inventor: Lee, Sanghyun, Gyeongsangbuk-do (KR)
(74) Representative: Botti, Mario

(57) **Abstract**

The present invention relates to a modified cell prepared by putting material into the cell without using delivery vehicle and to a cell composition containing the modified cell.

## Description

### Technical Field

The present invention relates to a modified cell prepared by putting material into the cell without using delivery vehicle and to a cell composition containing the modified cell.

More particularly, the present invention is directed to a modified cell prepared by putting material into the cell through a process by using a device which comprises: i) a first passage on which the cell passes; ii) a second passage on which said material passes and connected to the first passage at a position randomly selected between both ends of the first passage; and iii) an apparatus which applies pressure difference or electric potential difference on the first passage and the second passage, and the process comprises: i) a step of flowing a fluid containing the cell through the first passage and flowing the material through the second passage; and ii) a step for putting the material into the cell flowing through a site where the second passage is connected to the first passage by applying electric potential difference and/or pressure difference on the second passage.

### Backgroud of the Invention

Recently, various researches for new biomedical technology have been actively progressed by means of the fusion of biotechnology, electronic technology and nanotechnology which have been also remarkably developed lately.

Numerous attempts have been made to use patient's cells manipulated in vitro for medical treatment. Various R&D projects for new drug for next generation and for verifying the drug target by manipulating human cell, have been progressed.

The cell manipulation technologies have focused on the development of useful cellular therapeutics. Particularly, various exertions for cell therapy which utilizes the IPS (Induced Pluripotent Stem) cells induced by Yamanaka factor have been tried.

Yamanaka factor refer to four genes, Oct3/4, Sox2, cMyc and K1f4. The insertion of the four genes into the chromosome of cell by using the vector originated from virus, can transform the somatic cell which finished differentiation already into pluripotent stem cell which can differentiate into various somatic cells. IPS cell has been evaluated as an innovative technology which can overcome the ethics problem and productivity problem of embryonic stem cell, and can obviate also the limitations in differentiation capability of adult stem cell.

However, IPS cell cause a safety problem that the vector derived from virus inserted into live-cell together with Yamanaka factors. Also, in case of transplantation of the cell or tissue differentiated from IPS which contains the vector derived from virus into human body, there may be another problem that tumor risk is increased.

Therefore, new technologies for injecting various materials, such as, DNA, RNA, polypeptide, or nano-particle, directly into a cell without using delivery vehicle, have been required in order to develop new cellular therapeutics which can avoid the above risk caused by using of the viral vector.

In the conventional cell manipulation technologies which do not employ any delivery vehicle, the typical process is to damage the membrane of the cells by mechanical shear force, chemical treatment or by applying electric field and then to allow the material such as genes which exist in extra-cellular fluid to flow into the inside of the cell of which membrane damaged, and to expect the damaged cell membrane to be recovered by self-healing capacity of the cell.

A variety of cell modification techniques, such as particle bombardment, micro-injection and electroporation, have been developed. Except for the micro-injection, these techniques are based on bulk stochastic processes in which cells are transfected randomly by a large number of genes or polypeptides.

The disadvantage of the conventional bulk electroporation the most widely used process for transfection of cells is that the injected dose cannot be controlled.

Therefore, microfluidics-based electroporation has emerged as a new technology for individual cell transfection. The microfluidics-based electroporation offers several important advantages over the bulk electroporation, including lower poration voltages, better transfection efficiency and a sharp reduction in cell mortality.

In 2011, a nanochannel electroporation technology which expose a small area of a cell membrane positioned adjacent to a nanochannel to very large local electric field strength, was disclosed to the public (L. James Lee et al, "Nanochannel electroporation delivers precise amounts of biomolecules into living cells", Nature Nanotechnology Vol. 6, November 2011, www.nature.com/naturenanotechnology published online on October 16, 2011).

The nanochannel electroporation device comprises two micro channels connected by a nanochannel. The cell to be transfected is positioned in one microchannel to lie against the nanochannel, and other microchannel is filled with the agent to be delivered. The microchannel-nanochannel-microchannel design enables the precise placement of individual cells. One or more voltage pulses lasting milli-seconds is delivered between the two microchannels, causing transfection. Dose control is achieved by adjusting the duration and number of pulses.

By the way, the nanochannel electroporation device disclosed in the above prior art employs polydimethylsiloxane lid that covers microchannel and nanochannels made by polymeric resin through imprinting and formed over the chip substrate.

Therefore, the nanochannel electroporation (NEP) chip discribed in the article of Nature Nanotechnology, cannot avoid the chinks occurred between the polydimethylsiloxane lid and the imprinted layer of microchannels and nanochannels made by polymeric resin, because the bonding between the lid and the channel layer of which mechanical properties is different from each other, cannot be absolutely perfect.

Also, since the size stabilities of the polydimethysiloxane lid and the microchannels and nanochannels made by polymeric resin, are low, the bonding between the lid and the layer of channels cannot be perfect. Therefore, the chinks may easily occur between the lid and the layer of channels. The chinks allows the infiltration of the solution which causes the contamination of nanochannel electroporation chip and also generate various aberration of electric field and pressure difference applied for the transfer of cell between the channels and for injection of the transfection agent into the cell.

Therefore, new technology which can put various materials into individual cell quantitatively without using delivery vehicle and can control the amount of the material without such contamination or aberration, has long been anticipated in this technology field.

The inventor of the present application conceived a novel process for modifying an individual cell by putting material into the cell without using delivery vehicle by means of a device formed within one solid without any bonding in order to exclude the possibility of the occurrence of the chink intrinsically, and thereby, can obviate disadvantages of the prior art, such as the contamination and aberration caused by the chink.

The primary object of the present invention is to provide a modified cell prepared by putting material into the cell without using a delivery vehicle by means of the device formed within one solid, which comprises: a first passage on which the cell passes; a second passage on which the material passes and connected to the first passage at a position randomly selected between both ends of the first passage; and an apparatus which applies pressure difference or electric potential difference on the first passage and the second passage.

The another object of the present invention is to provide a cell composition which comprises the modified cell prepared by putting the material, such as, protein, peptide glycoprotein, lipoprotein, DNA, RNA, anti sense RNA, siRNA, nucleotide, ribozyme, plasmid, chromosome, virus, drug, organic compounds, inorganic compounds, hyaluronic acid, collagen, cell nucleus, mitochondria, endoplasmic reticulum, golgi body, lysosome, ribosome or nanoparticle, into the cell, and further comprises cell-culture medium and/or natural or artificial intercellular ground substance.

### Disclosure of invention

The object of the present invention can be achieved by providing a modified cell prepared by putting material into the cell through a process by using a device which comprises: i) a first passage on which the cell passes; ii) a second passage on which said material passes and connected to the first passage at a position randomly selected between both ends of the first passage; and iii) an apparatus which applies pressure difference or electric potential difference on the first passage and the second passage, and the process comprises: i) a step of flowing a fluid containing the cell through the first passage and flowing the material through the second passage; and ii) a step for putting the material into the cell flowing through a site where the second passage is connected to the first passage by applying electric potential difference and/or pressure difference on the second passage.

The material injected into the cell of the present invention may be protein, peptide glycoprotein, lipoprotein, DNA, RNA, anti sense RNA, siRNA, nucleotide, ribozyme, plasmid, chromosome, virus, drug, organic compounds, inorganic compounds, hyaluronic acid, collagen, cell nucleus, mitochondria, endoplasmic reticulum, golgi body, lysosome, ribosome or nanoparticle.

The another object of the present invention can be achieved by providing a cell composition which comprises the modified cell and cell-culture medium and/or natural or artificial intercellular ground substance which is selected from gelatin, cellulose, hyaluronic acid polymer, collagen, lipid, elastin, polysaccharide, pectin material or mixtures thereof.

The cell modified by putting material in the present invention, may be prokaryotic or eukaryotic cell. More particularly, the prokaryotic cell is bacteria or archaea. The eukaryotic cell is animal cell, insect cell or plant cell.

The animal cell modified in the present invention, may be somatic cell, reproductive cell or stem cell. More particularly, the somatic cell may be epithelial cell, muscle cell, nerve cell, fat cell, bone cell, red blood cell, white blood cell, lymphocyte or mucosa cell.

The reproductive cell modified in the present invention may be an egg cell or a sperm. The stem cell may be adult stem cell or embryonic stem cell. More particularly, the adult stem cell may be hematopoietic stem cell, mesenchymal stem cell, neural stem cell, fibroblast, liver fibroblast, retinoblast, adipose derived stem cell, bone marrow derived stem cell, umbilical cord blood derived stem cell, umbilical cord derived stem cell, placenta derived stem cell, amniotic fluid derived stem cell, peripheral blood vessel derived stem cell or amnion derived stem cell.

The device used for the preparation of the modified cell of the present invention is described in Korean patent applications Nos. 10-2014-0191302 and 10-2015-0178130. The process for preparation of the modified cell is described in Korean patent application Nos. 10-2014-0191302 and 10-2015-10-2015-0178183.

Microfluidics which had emerged in 2000 mainly deals with the analysis and manipulation of biomedical samples based on the micro-scale fluidic channel networks. The microfluidic chips have complex fluidic networks, where diverse biochemical surface treatments are engineered; electrochemical manipulations are performed; and pressure-driven or electro osmotic flow is driven to circulate the chip.

Thus, there are a lot of standards which the microfluidic chip should meet for the medical applications. And many of them can be ideally met with the incorporation of glass as the material of the microfluidic chip. This is because the glass has long been used and approved in the medical fields. Specifically, glass can well satisfy the biological compatibility, chemical resistance, electrical insulation, dimensional stability, structural strength, hydrophilicity, and transparency.

Nevertheless, incorporating glass as the material of microfluidic chips has difficulties and limitations originated from the etch-bond process such that the isotropic glass etching limits resolution, aspect ratio, and cross-sectional shape of the channels. In addition, the glass to glass bonding to complete the microfluidic chip fabrication is not only difficult and expensive, but it also radically limits to build true three-dimensional structures especially in nanoscales. Instead, PDMS silicon molding processes had wide spread owing to the fact that it is cheap, easy to replicate many times, and weak but simple bonding process to glass.

While the PDMS molding is great alternative for research purposes, it is still limited to be used for the medical purposes due to the low bio-compatibility, bad chemical resistance, dimensional instability, structural weakness, and incomplete bonding. The incomplete bonding can easily allow the current and fluid to leak along the bonding interfaces. Considering that improving the performance of the microfluidic operation is dependent upon increasing the press or the electric fields, PDMS molding would be incompatible to the medical grade microfluidic operation in many cases.

When cells are manipulated in microfluidic chips, the pressure and the electric potentials should not be limited to maximize the performance and the operational freedom due to the structural weakness and the incomplete bonding issue. The structural strength can be overcome by adopting rigid and stable solids as the base material with acceptable transparency such as glass, polymethylmethacrylate(PMMA), polycarbonate(PC), and so on. However, it is still required to significantly improve the glass-etch-bond process to take the huge advantages of glass in the microfluidic medical applications.

In the current glass-etch-bond processes, the lid glass on which microfluidic channel networks are etched is bonded to the flat bottom one by applying heat or plasma in dust-free conditions. This process is great for mass-producing two-dimensional microscale fluidic chips. However, realizing true tree-dimensional nanoscale structures by the glass-etch-bond process is significantly limited by the isotropic etching and the requirement for the glass-to-glass direct bonding.

The laser processing is thus promising, where the bonding process can be removed. Furthermore, research in the Univ. of Michigan, USA found that true three-dimensional nano-machining of glass is possible by incorporating the femtosecond laser pulses in 2004. Afterwards, femtosecond nanomachining has been developed, realizing the direct processing of true three-dimensional nanoscale structures in a single glass plate without bonding.

In most cases, it is much more convenient and efficient to prepare microscale two-dimensional channels based on the conventional etch-bond process and to process the rest of the nanoscale and three-dimensional structures by the femto-laser nanomachining. It is because the material removal of femto-laser nanomachining is inevitably very slow and localized, whereas the etching process can be effective for the large area processing.

The device used for the preparation of the modified cell of the present invention, is formed within one solid, such as, glass, thermoplastic polymers or thermosetting polymers, for example, polycarbonate, acrylics, epoxy resin or polyimide in order to exclude a possibility of occurrence of chink. The solid material is desirably selected from thermoplastic polymers, thermosetting polymers or glass. The transparency of the solid material employed for the device used for the preparation of the modified cell of the present invention, is desirably higher than 5%.

The first passage of the device used for the preparation of the modified cell of the present invention, has an inner tapered tube shape that inner diameter is reduced gradually from the both ends to middle section. Therefore, the inner diameters of both ends of the first passage are larger than those of the middle section of the first passage. Desirably, the inner diameters of both ends of the first passage is 10*µ*m to 200*µ*m and the inner diameters of middle section of the first passage is 3*µ*m to 150*µ*m.

The device used for the preparation of the modified cell of the present invention may comprise one or more of the second passage on which the material to be injected into a cell flows, in order to put several materials into a cell at one try. The inner diameter of the second passage is desirably 10nm to 1,000nm.

The cells contained in the first passage of the device used in the present invention, may flow by pressure difference or by electric potential difference between the both ends of the first passage. The electric potential difference between the both ends of the first passage is desirably 10 V to 1,000 V, more desirably 15 V to 500 V, most desirably 20 V to 200 V.

Also, the electric potential difference between the first passage and the second passage is desirably 0.5 V to 100 V, more desirably 0.8 V to 50 V, most desirably 1.0 V to 10 V.

The flow of the fluid containing the cell may effectively be controlled by adjusting the pressure difference or the electric potential difference applied on the first passage of the device used in the present invention during watching the movement of the cells in the first passage through microscope.

In addition, the amount of the material to be injected into a cell may be controlled by adjusting the electric potential difference between the first passage and the second passage of the present invention. Also, the amount of the material injected into a cell may be calculated i) by measuring the intensity of the fluorescent conjugated on the material to be injected or ii) by measuring the electric signal generated upon injecting the material into a cell

### Brief description of the drawings

Hereinafter, the modified cell of present invention will be described in greater detail with reference to the following examples. However, the examples are given only for illustration of the present invention and not to be limiting the process of present invention within the examples.
Figure 1 is a schematic diagram of the device used for the preparation of the modified cell of the present invention. Figure 1a shows the device for injecting material into a cell which has one material passage (the second passage), and figure 1b shows the device used for the present invention which has three second passages.
Figure 2 is a schematic diagram of the device used for the preparation of the modified cell of the present invention.
Figure 3 shows a three dimensional structure of the first passage and the second passage of the device used for injecting material into a cell (fig.3a), and fig. 3b is an enlarged diagram for the part which connects the first passage and the second passage.
Figure 4 is a schematic diagram which shows the process for injecting material into a cell, in Examples 2 to 5 of the present invention.
Figure 5 is the microscopic images for the device used for injecting material into a cell, prepared in Example 1 of the present invention.
Figure 6 is a photograph of external appearance of the device used for putting material into a cell.
Figure 7 is the microscopic images showing the process of injecting the red fluorescent protein into the human alveolar basal epithelial cell A549 in Example 2 of the present invention.
Figure 8 is the photographs magnifying the cell part in the process of injecting the red fluorescent protein into the human umbilical cord stem cell in Example 3 of the present invention.
Fig. 9 shows the photographs of fluorescence microscope of the proceedings of injecting RFP into the human placental stem cell in Example 4 of the present invention.
Fig. 10 shows the images of human alveolar basal epithelial cell A549, after the lapse of 12 hours from the injection of plasmid DNA(cy3) in Example 5 of the present invention.
Fig. 11 shows a disassembled perspective view of the laser beam machine employed for the processing of the device used for the present invention.

### Example 1: Process for forming the micro channels and nano channels of the device within one solid glass

Femto-laser pulses (Pharos, 4W, 190fs, frequency doubled 510nm, DPSS chirped pulse amplification laser system) had been focused on the single glass substrate through an objective lens (from 40× to 100×, N.A. from 0.5 - 1.3, Olympus & Zeizz), where the focus can move from the outside of the substrate into it. The glass substrate had been placed on the 3 axis linear nano-stage (100×100×100µm3, ±1nm, Mad City Labs, Inc., Madison, WI), by which the glass substrate had been able to be controlled in three-dimension against the focus with nanoscale accuracies.

As the focus of the femto-laser pulses had been controlled to move from the glass surface into it, the glass had been removed along the pathway of the focus. The pathways of the focus had been written as G-code to automatically machine true three-dimensional structures directly inside of the glass substrate. Thus, no glass-to-glass bonding had been required.

The entire process had been monitored by CCD camera. Although the minimum size of the femto-laser ablation of glass was found as 10nm, the setup of the example had been designed to have feature size around 200nm, and the feature size had been able to be controlled bigger or smaller than 200nm by adjusting the optical parameters and components. Machining true three-dimensional structures had been possible by using transparent material like glass.

### Example 2: Process of putting red fluorescence protein into human alveolar basal epithelial cell

RFP (dsRed fluorescence protein, MBS5303720) had been prepared by diluting to be 1mg/ml (solvent: PBS, Hyclone, SH30028.02, pH 7.4). A549 cells (human alveolar basal epithelial cells) had been subcultured using 10% FBS DMEM (high glucose) in an incubator (humidified 5% CO², 37 °C).

The subcultured cells had been separated using TrypLE (gibco). And the solution had been replaced with the 1 mM EDTA in D-PBS (gibco) solution. Debris had been filtered using 40 *µ*m cell strainer (BD), and then cells had been treated with Calcein-AM in an incubator (for 15 minutes, at 37°C). After the solution had been replaced with 1mM EDTA in D-PBS, A549 cell suspension solution of 2 x 10⁶ cells/ml concentration had been prepared using hemocytometer.

A549 cell suspension and RFP had been packaged in 1ml syringes, and each of them had been connected with silicon tubes to the inlets of the cell loading and the material loading channels. And the other sides of the cell loading and the material loading channels had been connected with silicon tubes to the PBS filled 1ml syringes.

By controlling all the syringes cells had been controlled to flow into the cell loading channels and each of them had been placed at the center of the cell loading channel where the material injection pathways were crossed.

Then, proper electric potentials had been applied to the both side of the material loading channel for 3 seconds to make the electric potential 1.76V along the material injection pathways. The electric potential had been measured by oscilloscope (VDS3102, Owon) and epifluorescent microscope (TE2000-U, 41-17Nikon) had been used to monitor cells and RFP injection process.

### Example 3: Process of putting red fluorescence protein into human umbilical cord tissue mesenchymal stem cell

RFP (dsRed fluorescence protein, MBS5303720) had been prepared by diluting to be 1mg/ml (solvent: PBS, Hyclone, SH30028.02, pH 7.4). UC-MSCs (Human Umbilical Cord Tissue Mesenchymal Stem Cells) had been taken from the CHA hospital at Boondang South Korea. UC-MSCs had been subcultured using MEM-alpha (Gibco), 10% FBS (Hyclone), 25 ng/ml FGF-4 (Peprotech), 1 ug/ml Heparin (Sigma) in an incubator (humidified 5% CO², 37 °C).

The subcultured cells had been separated using TrypLE (gibco). And the solution had been replaced with the 1 mM EDTA in D-PBS (gibco) solution. Debris had been filtered using 40 *µ*m cell strainer (BD), and then cells had been treated with Calcein-AM in an incubator (for 15 minutes, at 37°C). After the solution had been replaced with 1mM EDTA in D-PBS, UC-MSC suspension solution of 2 x 10⁶ cells/ml concentration had been prepared using hemocytometer.

UC-MSC suspension and RFP had been packaged in 1ml syringes, and each of them had been connected with silicon tubes to the inlets of the cell loading and the material loading channels. And the other sides of the cell loading and the material loading channels had been connected with silicon tubes to the PBS filled 1ml syringes.

By controlling all the syringes cells had been controlled to flow into the cell loading channels and each of them had been placed at the center of the cell loading channel where the material injection pathways were crossed.

Then, proper electric potentials had been applied to the both side of the material loading channel for 3 seconds to make the electric potential 1.45V along the material injection pathways. The electric potential had been measured by oscilloscope (VDS3102, Owon) and epifluorescent microscope (TE2000-U, 41-17Nikon) had been used to monitor cells and RFP injection process.

### Example 4: Process of putting red fluorescence protein into human placenta-derived mesenchymal stem cell

RFP (dsRed fluorescence protein, MBS5303720) had been prepared by diluting to be 1mg/ml (solvent: PBS, Hyclone, SH30028.02, pH 7.4). PD-MSCs (Human Placenta-derived Mesenchymal Stem Cells) had been taken from the CHA hospital at Boondang South Korea. PD-MSCs had been subcultured using MEM-alpha (Gibco), 10% FBS (Hyclone), 25 ng/ml FGF-4 (Peprotech), 1 ug/ml Heparin (Sigma) in an incubator (humidified 5% CO², 37 °C).

The subcultured cells had been separated using TrypLE (gibco). And the solution had been replaced with the 1 mM EDTA in D-PBS (gibco) solution. Debris had been filtered using 40 *µ*m cell strainer (BD), and then cells had been treated with Calcein-AM in an incubator (for 15 minutes, at 37°C). After the solution had been replaced with 1mM EDTA in D-PBS, PD-MSC suspension solution of 2 x 10⁶ cells/ml concentration had been prepared using hemocytometer.

PD-MSC suspension and RFP had been packaged in 1ml syringes, and each of them had been connected with silicon tubes to the inlets of the cell loading and the material loading channels. And the other sides of the cell loading and the material loading channels had been connected with silicon tubes to the PBS filled 1ml syringes.

By controlling all the syringes cells had been controlled to flow into the cell loading channels, and each of them had been placed at the center of the cell loading channel where the material injection pathways were crossed.

Then, proper electric potentials had been applied to the both side of the material loading channel for 5 seconds to make the electric potential 0.87V along the material injection pathways. The electric potential had been measured by oscilloscope (VDS3102, Owon) and epifluorescent microscope (TE2000-U, 41-17Nikon) had been used to monitor cells and RFP injection process.

### Example 5: Process of putting plasmid DNA(cy3) into human alveolar basal epithelial cell

Plasmid DNA(MIR7904, Mirus) had been prepared by diluting to be 10*µ*g 20*µ*ℓ. A549 cells (human alveolar basal epithelial cells) had been subcultured using 10% FBS DMEM (high glucose) in an incubator (humidified 5% CO², 37 °C).

The subcultured cells had been separated using TrypLE (gibco). And the solution had been replaced with the 1 mM EDTA in D-PBS (gibco) solution. Debris had been filtered using 40 *µ*m cell strainer (BD), and then cells had been treated with Calcein-AM in an incubator (for 15 minutes, at 37°C). After the solution had been replaced with 1mM EDTA in D-PBS, A549 cell suspension solution of 2 x 10⁶ cells/ml concentration had been prepared using hemocytometer.

A549 cell suspension and RFP had been packaged in 1ml syringes, and each of them had been connected with silicon tubes to the inlets of the cell loading and the material loading channels. And the other sides of the cell loading and the material loading channels had been connected with silicon tubes to the PBS filled 1ml syringes.

By controlling all the syringes cells had been controlled to flow into the cell loading channels, and each of them had been placed at the center of the cell loading channel where the material injection pathways were crossed.

Then, proper electric potentials had been applied to the both side of the material loading channel for 2 seconds to make the electric potential 1.0V along the material injection pathways. The electric potential had been measured by oscilloscope (VDS3102, Owon) and epifluorescent microscope (TE2000-U, 41-17Nikon) had been used to monitor cells and RFP injection process.

After the injection of Plasmid DNA into A549 cells, the harvested cells had been distributed in the 96 well plate with 200 *µ*ℓ culture fluids. After culturing for 12 hours (humidified 5% CO², 37 °C), red fluorescence inside of the cells had been induced to confirm that the plasmid DNA had been successfully expressed.

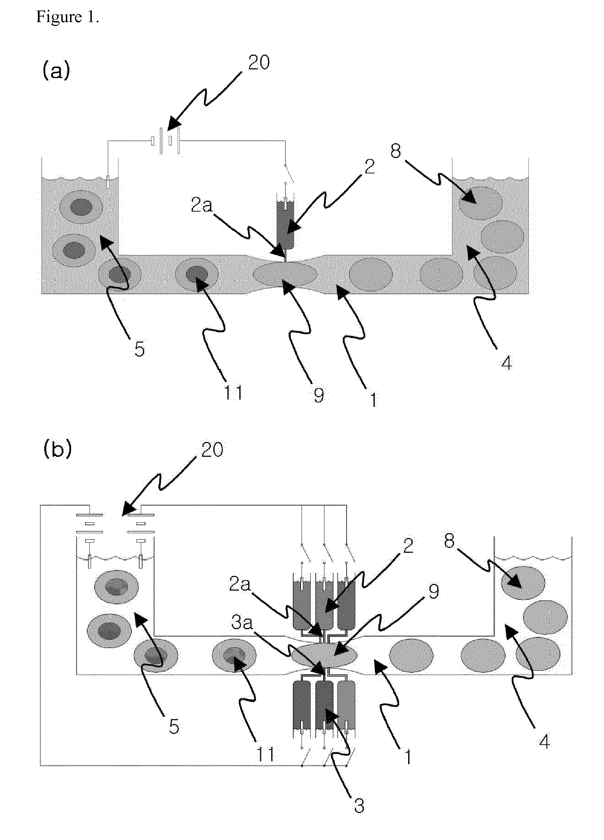

For reference to Fig. 1, the device used in the present invention has one material passage (the second passage, Fig.1a) or has three second passages (the second passage, Fig.1b) are shown.

In the Fig. 1, the cell (8) to be injected with the material moves through the first passage. The electrical potential difference occurs between the second passage (2) which injects the material and the first passage by the external power (20). The material (2a) is injected to the cell (9) by the electrical potential difference between the cell and the second passage when the cell (8) passes the narrowed middle section of the first passage. The cell which has been injected with material (11) is moved to the cell draw off passage (5) one after another.

In the Fig. 1b, the device used in the present invention injecting six (6) materials by employing the six (6) second passage is represented. Using the device illustrated in Fig. 1b, it is possible to put six materials into a cell at a time.

In Figs. 1a and 1b, it is possible to control the amount of each material to be put into an individual cell by adjusting the electrical potential difference between the first passage and the each second passage.

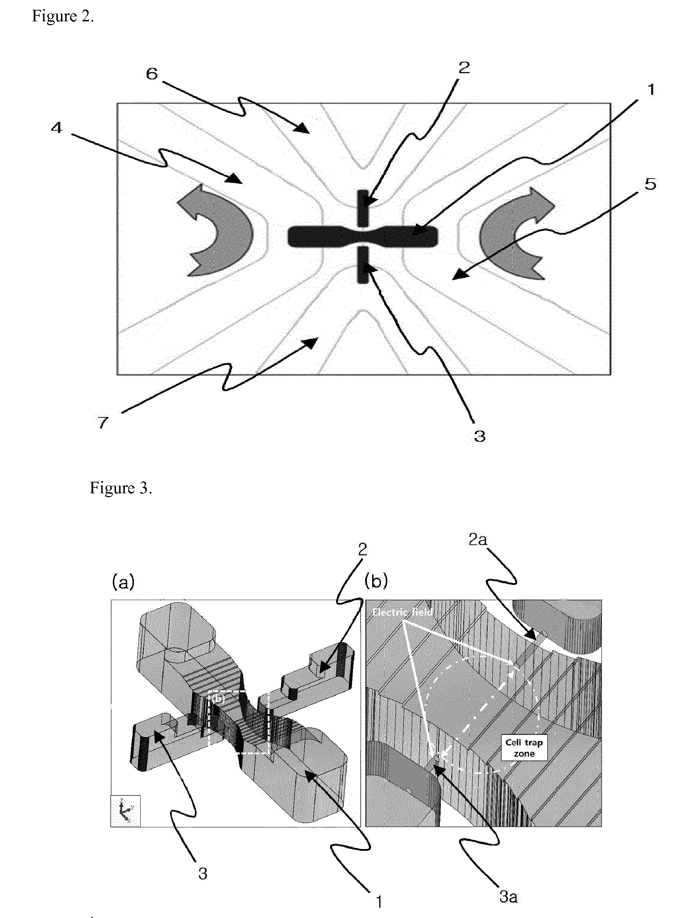

In the Fig. 2, there are the outflow and inflow channels (4) of the solution containing the cell, the outflow (7) and inflow (6) channels of material, the passage (1) and channel (5) which take back the cell that has been injected with material, the first passage (1) which has a shape of narrowed middle section, and the two second passages (2,3) which is connected to the middle section of the first passage (1).

During the cell passes through the narrowed portion of the middle section of the first passage (1), the cell is inserted and held on the inner wall of the middle section of the first passage. By this close contact between the cell and the wall of the middle section of the first passage (1), the drop of the electrical potential difference between the first passage (1) and second passage (2, 3) is minimized. The cell that has been injected with the material can easily be moved to the widened portion of the other side of the first passage.

The second passage (2, 3) plays a role as like an injection needle. That is, the charge focused on the second passage (2a, 3a) by an external electrical power provides the function of boring the cell membrane (or cell wall) of the individual cell which is closely contacted to the second passage (2a, 3a), and the driving force for injecting the material into the cell through the pore formed by the boring.

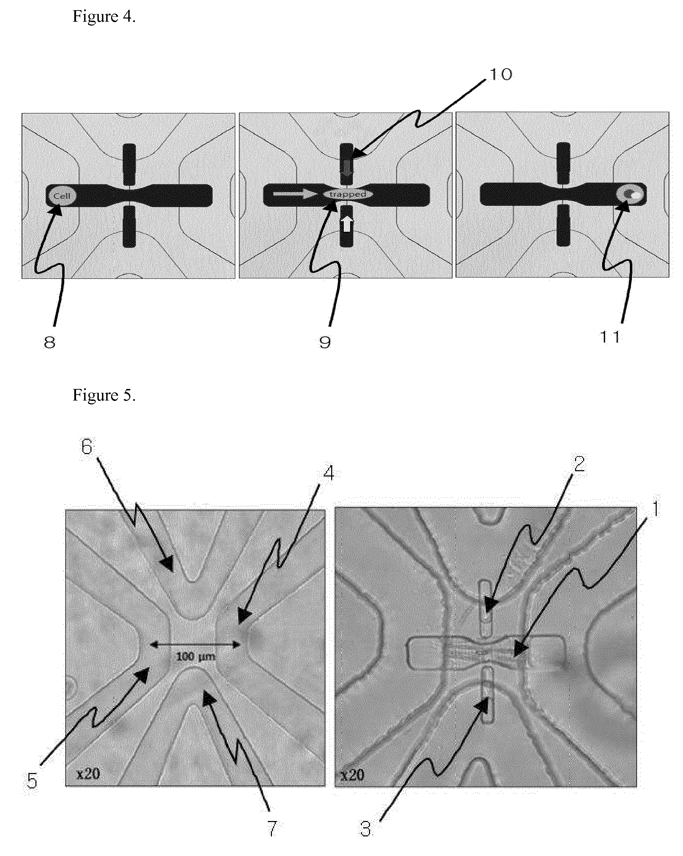

By applying pressure difference (for example by using a pump) or by applying electrical potential difference (for example by using external electrical potential with direct current or alternating current) to the inflow and outflow channels (Fig. 2, 4) of solution containing the cells (8 of Fig.1a and Fig.1b), the cell moves through the first passage (1). After the injection of material into the cell, the cell moves to the other side of the first passage (5 of Fig.1a and 1b).

The inflow and outflow channels (6, 7 of Fig.2) for material to be injected, intake and discharge the material by pressure difference or electrical potential difference between the inflow channel and outflow channel. Also, during the individual cell is closely contacting with the second passage in the narrowed middle section of the first passage, micro pore is generated on the cell membrane (or cell wall) and then the material is injected into the cell by the electric potential difference.

As represented in Fig.3, the second passages (2,3) are connected to the narrowed middle section of the first passage (1).

The Fig. 4 illustrates a process of injecting two kinds of materials into a single cell. The two materials are injected (11) into the cell trapped in the narrowed middle section of the first passage (9) through which the second passages (10) are connected, and then the cell injected with the material is retrieved through the outflow channel connected to the first passage.

The left microscopic image of Fig.5 shows the inflow and outflow channel (5) of solution containing the cell, the inflow and outflow channels (6, 7) of material which will be injected into the cell, and the outflow channel (4) of retrieving the cell into which the material has been injected.

In addition to the left image in Fig.5, the right image of Fig.5 shows the first passage (1) having the narrowed middle section and connected at the both of ends to the inflow and outflow channel (5) and the channel (4) of retrieving the cell into which the material has been injected. The microscopic images of Fig.5 also show the two second passage (2, 3) formed within the glass and connected to the narrowed middle section of the first passage.

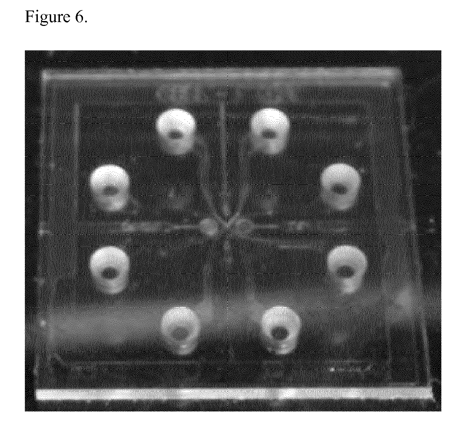

Fig.6 is a photograph of external appearance of the device used in the present invention for putting material into a cell.

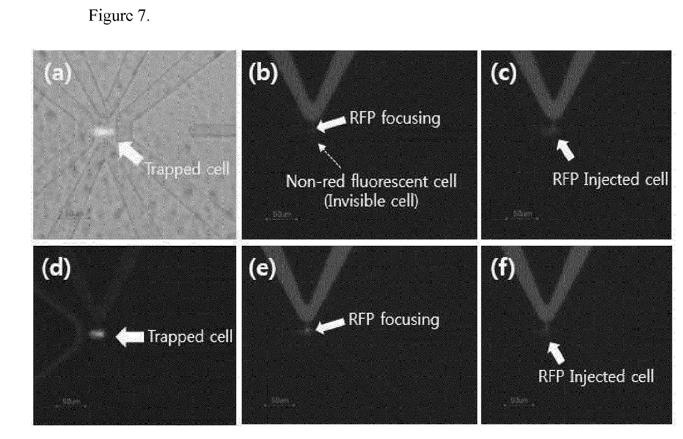

Fig.7 shows the photographs of fluorescence microscope of the proceedings of injecting the red fluorescence protein (RFP) into the human alveolar basal epithelial cell A549 in Example 2. The aliveness of the human alveolar basal epithelial cell after injection with the red fluorescence protein, was confirmed by means of the test of ascertaining green fluorescence from the human alveolar basal epithelial cell treated with Calcein AM(fluorescent dye).

Fig.7a shows that the live (green fluorescence) A549 cell locates in the middle section of the first passage. Fig.7b shows that when the electrical potential difference is applied between the first passage and the second passage, RFP which has been moved along the second passage, starts to be injected into the A549 cell (red fluorescent). Fig.7c shows the fact that RFP is being injected into the A549 cell (red fluorescent) certainly. Fig.7d shows that the A549 cell (green fluorescent) is alive after the RFP injection. Figs.7e and 7f show that RFP has been injected successfully into the A549 cell after the repeating two times the above procedure.

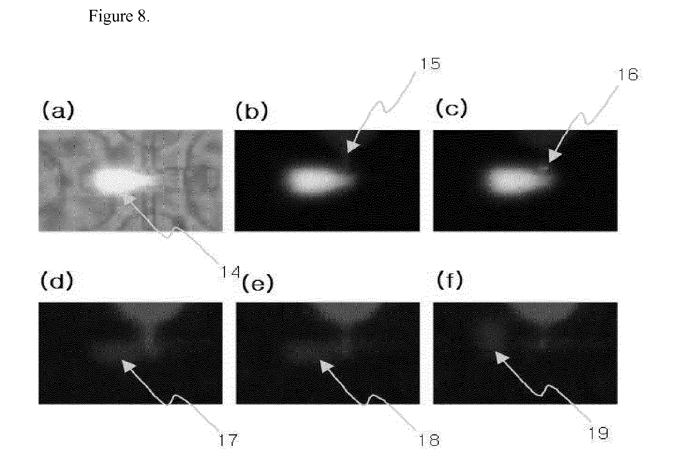

Fig.8 shows the photographs of fluorescence microscope of the proceedings for injecting RFP into the human umbilical cord stem cells. As described in the above explanation of Fig.7, the aliveness of the human umbilical stem cell was confirmed by using the Calcein AM.

Fig.8a shows that the human umbilical cord stem cell located in the middle section of the first passage is alive by means of the green fluorescence (14). Fig.8b shows that the second passage is stocked with RFP by means of the red fluorescence (15), and that injection of RFP into the human umbilical cord stem cell is prepared well by means of the green fluorescence. Fig. 8c shows that the injection of RFP is starting upon the application of the electrical field on the passages by means of the appearance of the brighter red fluorescence (16). Fig. 8d shows that the umbilical cord stem cell is moving to the exit side of the first passage by means of the red fluorescence (17). Fig.8f shows that umbilical cord stem cell is totally discharged from the first passage by means of the red fluorescence (19).

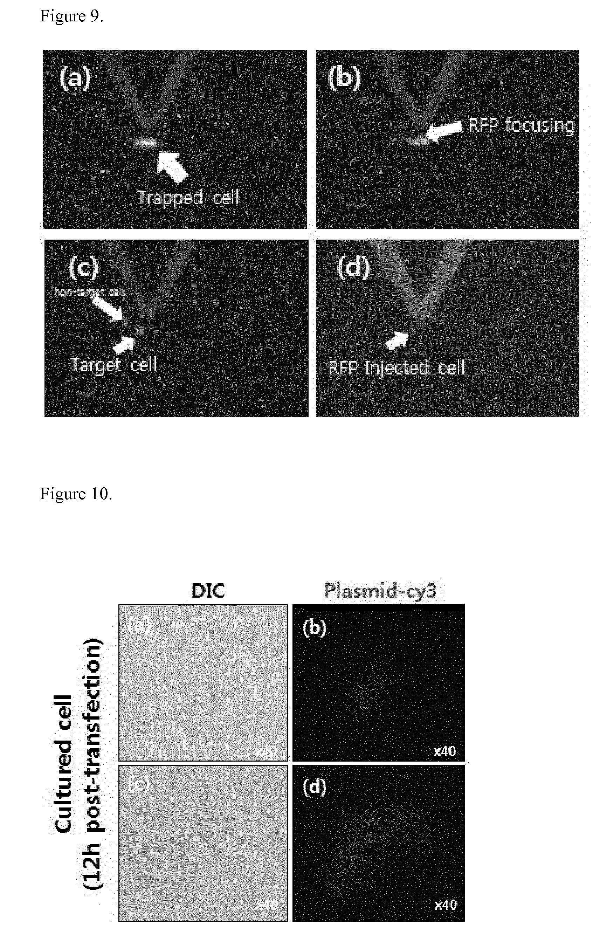

Fig. 9 shows the photographs of fluorescence microscope of the proceedings of injecting RFP into the human placental stem cell. As described in the above explanation of Fig.7, the aliveness of the human umbilical stem cell was confirmed by using the Calcein AM.

Fig. 10 shows the images of human alveolar basal epithelial cell A549 after the lapse of 12 hours from the injection of plasmid DNA (cy3) described in Example 5.

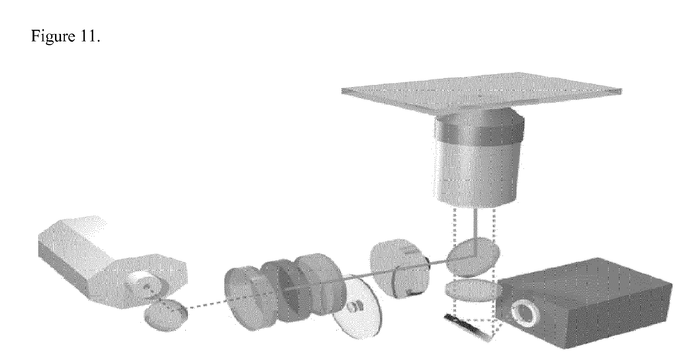

Fig. 11 shows a disassembled perspective view of the laser beam machine employed for the processing of the device used in process of the present invention.

### Advantageous effects

As explained above, for the preparation of the modified cell of the present invention, various materials, such as protein, gene, plasmid, drug, or nanoparticle can be putted into an individual cell. Particularly, the amount of the material putted into the single cell can be controlled by adjusting the electrical potential difference. Therefore, the modified cell can be used for a great variety of cellular therapeutics including IPS stem cells.

The foregoing examples 1 to 5 and the description of Figs. 1 to 11 for the preferred embodiments should be taken as illustrating, rather than as limiting the present invention as defined by the claims.

As will be readily appreciated by a person skilled in the art, numerous variations and combinations of the features set forth above can be utilized without departing from the present invention as set forth in the claims. Such variations are not regarded as a departure from the spirit and scope of the invention, and all such variations are intended to be included within the scope of the following claims.

## Claims

1. A modified cell prepared by putting material into the cell through a process by using a bonding-less device which comprises:
i) a first passage on which the cell passes;
ii) a second passage on which said material passes and connected to the first passage at a position randomly selected between both ends of the first passage; and
iii) an apparatus which applies pressure difference or electric potential difference on the first passage and the second passage,
and the process comprises:
1) a step of flowing a fluid containing the cell through the first passage and flowing the material through the second passage; and
2) a step for putting the material into the cell flowing through a site where the second passage is connected to the first passage by applying electric potential difference and/or pressure difference on the second passage.

2. The modified cell according to Claim 1, wherein the material is selected from the group consisting of protein, peptide glycoprotein, lipoprotein, DNA, RNA, anti sense RNA, siRNA, nucleotide, ribozyme, plasmid, chromosome, virus, drug, organic compounds, inorganic compounds, hyaluronic acid, collagen, cell nucleus, mitochondria, endoplasmic reticulum, golgi body, lysosome, ribosome and nanoparticle.

3. The modified cell according to Claim 1, wherein the cell is prokaryotic or eukaryotic cell.

4. The modified cell according to Claim 3, wherein the prokaryotic cell is bacteria or archaea.

5. The modified cell according to Claim 3, wherein the eukaryotic cell is selected from the group consisting of animal cell, insect cell and plant cell.

6. The modified cell according to Claim 5, wherein the animal cell is selected from the group consisting of somatic cell, reproductive cell and stem cell.

7. The modified cell according to Claim 6, wherein the somatic cell is selected from the group consisting of epithelial cell, muscle cell, nerve cell, fat cell, bone cell, red blood cell, white blood cell, lymphocyte and mucosa cell.

8. The modified cell according to Claim 6, wherein the reproductive cell is an egg cell or a sperm.

9. The modified cell according to Claim 6, wherein the stem cell is adult stem cell or embryonic stem cell.

10. The modified cell according to Claim 9, wherein the adult stem cell is selected from the group consisting of hematopoietic stem cell, mesenchymal stem cell, neural stem cell, fibroblast, liver fibroblast, retinoblast, adipose derived stem cell, bone marrow derived stem cell, umbilical cord blood derived stem cell, umbilical cord derived stem cell, placenta derived stem cell, amniotic fluid derived stem cell, peripheral blood vessel derived stem cell and amnion derived stem cell.

11. A cell composition containing a modified cell prepared by putting material into the cell through a process by using a bonding-less device which comprises:
i) a first passage on which the cell passes;
ii) a second passage on which said material passes and connected to the first passage at a position randomly selected between both ends of the first passage; and
iii) an apparatus which applies pressure difference or electric potential difference on the first passage and the second passage,
and the process comprises:
1) a step of flowing a fluid containing the cell through the first passage and flowing the material through the second passage; and
2) a step for putting the material into the cell flowing through a site where the second passage is connected to the first passage by applying electric potential difference on the second passage.

12. The cell composition according to Claim 11, wherein the composition comprise cell-culture medium.

13. The cell composition according to Claim 11, wherein the composition comprises natural or artificial intercellular ground substance.

14. The cell composition according to Claim 13, wherein the intercellular ground substance is selected from the group consisting of gelatin, cellulose, hyaluronic acid polymer, collagen, lipid, elastin, polysaccharide, pectin material and mixtures thereof.
